# EUROPEAN PATENT SPECIFICATION

(11) **EP 1 514 528 B1**
(45) Date of publication and mention of the grant of the patent: **11.04.2007**
(21) Application number: 04028919.1
(22) Date of filing: 11.05.2000
(51) Int. Cl.: A61F 5/441

(54) **Venting/filter assembly for venting flatus gasses**
Entlüftungs-/Filteranordnung zum Entlüften von Blähungen
Dispositif de dégazage et filtration pour le mise à l'air de gaz intestinaux

(30) Priority: 11.05.1999 DK 64799
(43) Date of publication of application: 16.03.2005
(62) Divisional of application: 00610045.7
(73) Proprietor: Dansac A/S, 3480 Fredensborg (DK)
(72) Inventor: Villefrance, Tine, 2730 Herlev (DK)
(74) Representative: Nielsen, Henrik Sten

(56) References cited:
- EP-A- 0 443 728
- EP-A- 0 516 094
- WO-A-83/02890
- GB-A- 2 149 306
- GB-A- 2 296 660
- US-A- 4 778 601
- US-A- 5 306 264
- US-A- 5 417 678
- US-A- 5 643 234

## Description

The present invention relates to a filter/venting assembly for use in a bag or pouch for receiving discharge from the human body, said bag comprising a distal wall and a proximal wall, each of a gas and liquid impermeable, heat sealable thermoplastic material and having the edges thereof sealed together and defining the interior of the bag between the walls, the proximal wall having a stomal aperture, and one of the walls having a gas venting aperture spaced from the stomal aperture, the filter assembly for de-odorizing gas vented through the gas venting aperture being adapted for being arranged such that all the gas exiting the bag through the venting aperture flows through the filter and an intermediate barrier membrane arranged intermediate the filter and the interior of the bag such that all the gas exiting the bag through the filter flows through the barrier membrane and for preventing liquids and solids in the interior of the bag from contacting the filter while allowing gas to flow through the barrier membrane from the interior of the bag to the filter. The closest prior art is US-A-5306264, which defines the preamble of claim 1.

Bags or pouches for the use of colostomy and ileostomy patients require a very efficient and reliable venting of flatus gasses combined with a highly effective de-odorization of the gasses before being released to the surroundings. Filters of various kinds are known for de-odorizing the vented flatus gasses. A recurring problem in connection with many of the known filter/venting arrangements is that the filter becomes clogged by liquid/slurry/solids present in the material discharged to the bag thereby giving rise to an unacceptable build-up of pressure in the bag during use. This problem is particularly serious for ileostomy patients because of the relatively liquid character of the discharged material.

Various different solutions to this problem have been proposed comprising the use of a barrier membrane interposed between the filter and the discharged material in the bag. The barrier membranes proposed are in many instances stated to be impermeable to liquids, permeable to gasses and having a liquid repelling surface facing the discharged material without dwelling on the more detailed characteristics necessary to obtain this in practice in a reliable and cost-effective manner.

As the bags typically are replaced daily or more often, the cost-effectiveness of the bags is very important while no compromises as regards the reliability of i.a. the venting and de-odorizing of the flatus gasses are acceptable. Therefore, any improvements increasing the reliability of the bag without substantially increasing the price, not to mention while decreasing the price, are extremely desirable and render any bag thus improved far superior to the prior art bags.

EP 0 443 728 B1 discloses a bag of the type in reference wherein the barrier membrane interposed between the filter and the interior of the bag comprises a thermoplastic, gas permeable, heat sealable or weldable film laminated together with a liquid impermeable, gas permeable sheet of polytetrafluorethylene (PTFE). As the PTFE sheet is not heat sealable, the heat sealable or weldable film is necessary to heat seal or weld the membrane to an inner surface of the bag in a region surrounding the filter and venting aperture. The liquid repelling characteristics of PTFE are hereby exploited, but at the price of having to construct a laminate partly to reinforce the relatively fragile PTFE sheet and partly to allow a practical and reliable heat sealing of the membrane to the bag wall.

Apart from the complication and expense of having to laminate the two materials together and of the relatively expensive PTFE sheet, this solution is prone to delamination of the membrane with the consequent risk of rupture of the relatively fragile PTFE sheet and subsequent failure of the venting effect due to clogging of the filter by non-gaseous material passing through the ruptured region of the sheet.

The filter element disclosed in EP 0 443 728 B1 is in the form of a plane pad having two opposed surfaces and an edge surface and of the type where the flatus gasses flow through the pad in a direction substantially transverse to the opposed surfaces thereby affording a relatively low utilization of the filtering capability of the filter material because of short contact time between gasses and filter material and uneven flow rates through the different regions of the pad owing to unavoidable differences in flow resistance because of uneven thickness, uneven density and so on.

GB-A-2 059 797 discloses an odor absorbing gas venting filter assembly for being adhered to the inner wall of a collection bag of the type in reference. A barrier membrane is interposed between the filter pad and the interior of the bag, said barrier membrane being either a laminate or a cellulosic material coated with a water resistant layer and optionally supplemented with another film of various types. This filter assembly is relatively complicated to manufacture and is not reliable because of the inherent problems of lamination and coating as well as the fragile character of the cellulosic material. The flow path of the gasses through the filter pad is such that the filtering capability of the filter material is badly utilized.

A main object of the invention is to provide a filter/venting assembly of the type in reference that is reliable in use and relatively inexpensive and uncomplicated to manufacture and whereby the disadvantages of the known assemblies described above have been eliminated or substantially reduced.

According to the invention, this object is achieved by the features specified in the characterizing part of claim.

In addition to the problem of venting and de-odorizing the flatus gasses, it is highly desirable to remove at least some and preferably substantially all of the bacteria present in the flatus gasses before venting same. The applicant is not aware of any effort to deal specifically with this objective.

According to the invention, this object is attained by said film having a porosity substantially only allowing passage through the barrier membrane of particles in said gas flow having a maximum dimension smaller than 2 micrometres, preferably 1.9 micrometres, more preferably 1.8 micrometres, even more preferably 1.7 micrometres, further preferably 1.6 micrometres, further preferably 1.5 micrometres, further preferably 1.4 micrometres, further preferably 1.3 micrometres, further preferably 1.2 micrometres, further preferably 1.1 micrometres, further preferably 1.0 micrometres, further preferably 0.9 micrometres, further preferably 0.8 micrometres, further preferably 0.7 micrometres, further preferably 0.6 micrometres, further preferably 0.5 micrometres.

As most of the bacteria present in the human intestine or feces are larger than approx. 1 micrometre, many or all of said bacteria will be retained by the barrier membrane with a porosity as indicated above.

In the currently preferred embodiment, said film has a porosity substantially only allowing passage through the barrier membrane of particles in said gas flow having a maximum dimension smaller than 0.45 micrometres. Hereby, practically all human intestinal or feces bacteria are prevented from exiting the bag in the flow of flatus gasses. Such a membrane may be supplied by Millipore Corporation, Bedford, Massachusetts and according to US Patent No. 4,778,601.

Although constrained by the necessary total flow rate through the filter/venting arrangement that requires a certain maximum flow resistance per unit of surface area of the barrier membrane, the flow-through area of the barrier membrane may be so large that the porosity of the barrier membrane substantially only allows passage through the barrier membrane of particles in said gas flow having a maximum dimension smaller than 0.3 micrometres, preferably 0.2 micrometres. Hereby at least a substantial part of the human intestinal viruses are prevented from exiting the bag in the flow of flatus gasses. Such a membrane may also be supplied by Millipore Corporation.

It is also important for the cost-effectiveness and wearing comfort as well as the attractiveness of the filter/venting arrangement that the dimensions of the filtering element itself be kept to a minimum while providing for efficient and substantially constant filtering effect over a time period of typically at least 24 hours.

A filter/venting arrangement providing for an effective, reliable, relatively sterile and relatively inexpensive solution to these combined problems is therefore highly desirable.

In the preferred embodiment of the invention, the filter comprises a pad, a first surface of the pad facing the barrier membrane being covered by a gas impermeable film, preferably bonded thereto, such that the gas flow path through the filter pad extends from or to the circumferential edge thereof, the filter pad comprising at least two mutually superimposed layers of textile being highly, preferably nearly 100%, carbonized, each layer being bonded to an adjacent layer by means of a gas permeable adhesive film such as porous ethylene vinyl acetate (EVA).

It has turned out that a filter having this configuration is very superior to the filters currently in use in connection with pouches of the type in reference because the amount of activated carbon per unit of volume is at a maximum and the superimposition of at least two layers bonded by EVA affords a relatively long contact time between flatus gasses and filter material while substantially the entire mass of the filter is contacted by the flatus gasses with substantially the same flow intensity.

According to the invention, spacing means may be arranged between the filter and the barrier membrane so as to ensure a space therebetween for allowing a flow of gas in said space.

Hereby, it is ensured that as much as possible of the entire area of the barrier membrane available for passage of flatus gasses is utilized therefor by ensuring that abutment of the barrier membrane against the filter or the third film bonded to the filter does not limit flow of gasses through the barrier membrane.

The invention will now be explained more in detail and by way of example with reference to the drawings, wherein:
Figure 1 shows a planar view of the currently preferred embodiment of a bag according to the invention seen from the distal wall, wherein a venting aperture is arranged,
Figure 2 shows a diagrammatical cross-sectional view of the upper region of the bag in Figure 1 having a filter assembly package adhered to the inner surface of the distal wall,
Figure 3 shows an enlarged diagrammatical cut away cross-sectional view of the filter assembly in Fig. 1 indicating the flow of flatus gas,
Figure 4 shows a diagrammatical cross-sectional view of the upper region of an alternative bag having an alternative filter assembly package adhered to the outer surface of the distal wall,
Figure 5 shows an enlarged diagrammatical cut away cross-sectional view of the filter assembly in Fig. 4 indicating the flow of flatus gas,
Figure 6 shows a view corresponding to Figs. 2 and 4 of a double filter assembly having filter packages adhered to both the outer and inner surface of the distal wall of a bag.
Figures 7-10 show four alternative embodiments of a filter assembly according to the invention comprising four different embodiments of spacer means between the filter pad and the barrier membrane, and
Figure 11 shows a diagrammatical schematical elevational view of an application apparatus for producing a filter assembly according to the invention.

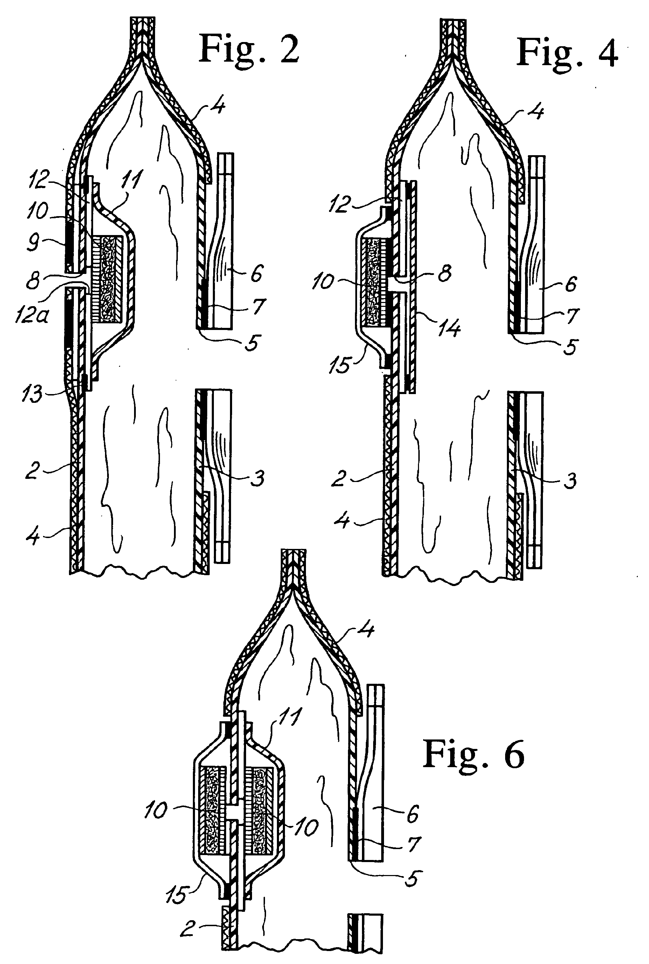

It should be noted that for the sake of clarity and illustration of the constructive details, the relative dimensions of the elements in Figs. 2-11 are not correct, and the shapes and locations of the various elements relative to one another are distorted relative to reality where the films and elements conform much more to one another than as depicted.

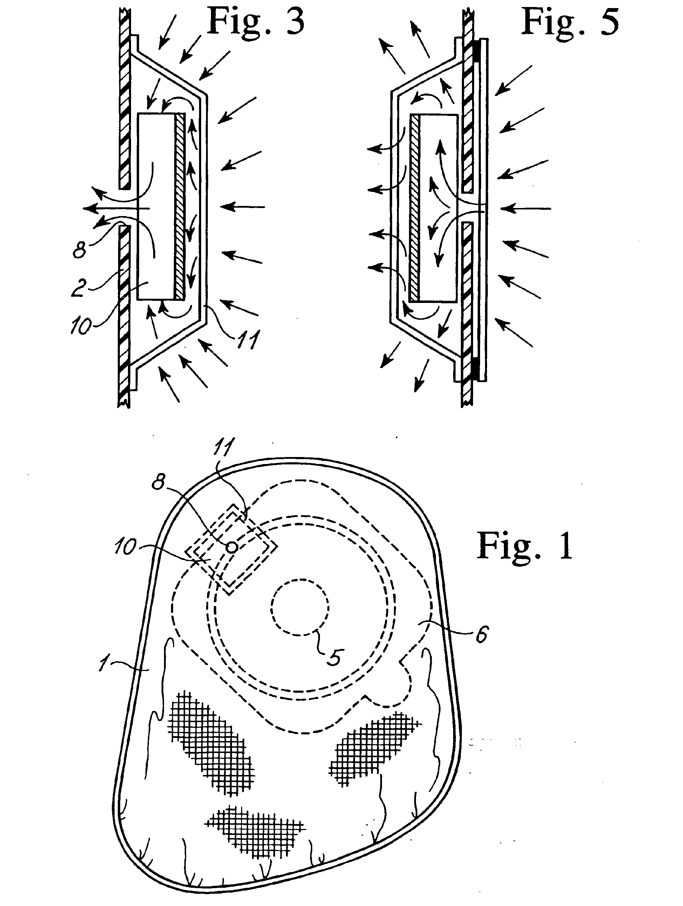

Referring now to Figs. 1-3, a bag or pouch 1 is defined by two bag sheets heat sealed or welded together at the edges thereof and constituting a distal wall 2 and a proximal wall 3 of the bag 1. The bag sheets are made of five layers of film with a PVDC film sandwiched between two pairs of preferably EVA and/or PE film such that suitable gas and liquid impermeable heat sealable thermoplastic walls 2 and 3 are obtained.

A backing layer 4 of soft linen, felt or the like is attached to the outer surface of the distal and proximal walls 2 and 3 for the comfort of the user of the bag.

The proximal wall 3 is provided with a stoma aperture 5 surrounded by a skin friendly adhesion plate 6 for adhering the bag 1 to a patient and heat sealed at 7 to the proximal wall 3. The distal wall 2 is provided with a venting aperture 8 for venting flatus gasses from the interior of the bag 1 to the surroundings.

A heat sealed area indicated at 9 is provided in the backing layer 4 surrounding the venting aperture 8 so as to provide a suitably smooth surface for temporarily adhering a tear-off tab for closing the venting aperture 8 for preventing flow of water into the bag 1 through the aperture 8 when a patient wearing the bag 1 takes a bath.

A filter assembly package or unit comprises an activated carbon filter pad 10 sandwiched between a barrier membrane 11 of ultra high molecular weight polyethylene (UPE) and an approx. 100 micrometres thick support film 12 of polyethylene with 10 % EVA having a venting aperture 12a with a diameter of approx. 5 mm. The filter assembly package is heat sealed to the inner surface of pouch wall 2 at 13.

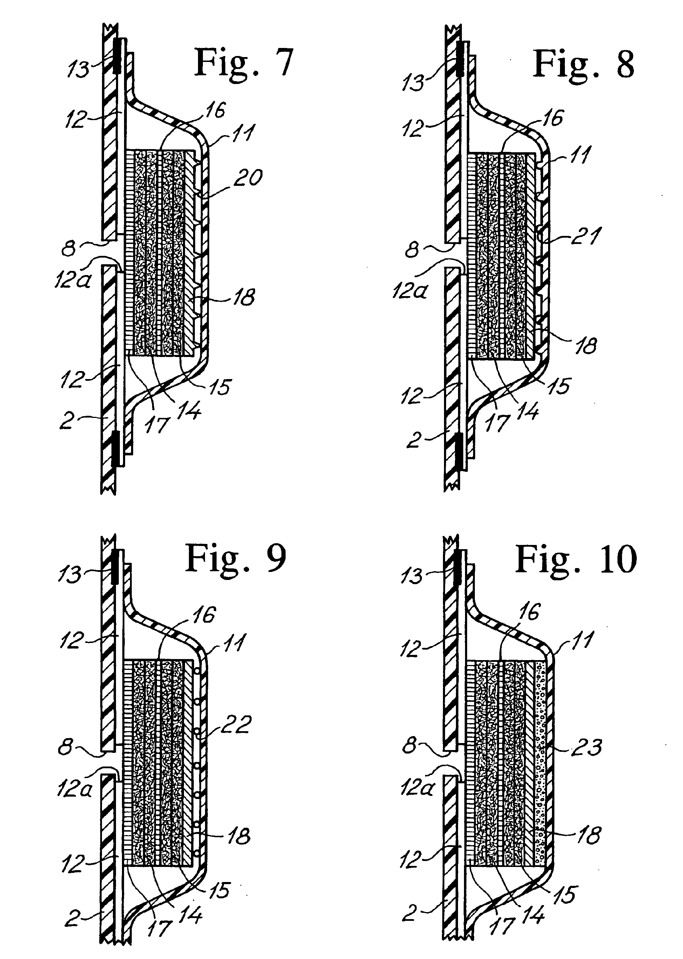

Referring now to Figs. 7-10, the filter pad 10 is a 20x20 mm² 100% active charcoal filter of the type FM7/250 supplied by Charcoal Cloth, UK having a thickness of approx. 1.3 mm. The charcoal textile consists of carbonized, woven viscose rayon textile. The carbonization takes place by means of a non-stochiometric process (low oxygen atmosphere to avoid burning of the material) at a temperature of 800-1000 degrees Celsius. The textile material of the pad consists of four layers interwoven in pairs, the pairs of layers 14 and 15 being adhered to one another by means of a gas permeable layer 16 of adhesive of polyurethane or EVA configured as a porous net.

The charcoal filter is impregnated with a 5% w/w solution of copper so as to obtain a more rapid and better adsorption of flatus gasses as well as with a 5% w/w solution of fluor to bind/retain the carbon particles in the material i.a. to avoid carbon dust.

The charcoal layers 14 and 15 are laminated to films 17 and 18, respectively, forming opposed outer surfaces of the filter pad. The film 17 is gas permeable and consists of a PE nonwoven material adhered to the charcoal layer 14 by means of an EVA adhesive configured as a porous net, while the film 18 is gas impermeable and consists of a material containing PVDC adhered to the charcoal layer 15 by means of an EVA adhesive.

The charcoal filter is not or only partly liquid repelling in itself, but it is extremely efficient as a de-odorizing filter. The carbonised textile layers contain 0.16-0.18 g carbon (dry) and according to the modified British Standard testing method BS 7120, the filter adsorbs more than 30-40 I gas mixture, 30 ppm H2S in N2. A corresponding test with (CH3)2S shows that the filter can adsorb more than 40-60 I gas mixture, 30 ppm (CH3)2S in N2. The filter has a back pressure of max. 4.5 mBar with a gas flow therethrough of 100 ml/min at a pressure of 0.1 Bar. This low back pressure allows the flatus gasses to flow relatively quickly through the filter, thus avoiding ballooning of the bag 1.

The ultra high molecular weight polyethylene barrier membrane 11 is supplied by Millipore Corporation, Bedford, Mass., USA and is according to US patent No. 4,778,601. It has a pore volume of 70-80% and such that substantially all particles in the flatus gasses with a maximum dimension of 0.45 micrometres cannot pass through the barrier membrane, i.e. are retained thereby such that such particles cannot pass to the filter pad 10 from the interior of the pouch 1. The thickness of the barrier membrane 11 is 148 micrometres plus/minus 20 micrometres. The size is approx. 32x32 mm. The Flow Time is in the range of approx. 150 sec/500 ml to approx. 300 sec/500 ml in 100% IPA (applied to a circular membrane with a diameter of 47 mm at a pressure of 14.2 psi). Porosity determined by the Bubble Point Method is in the range of approx. 15 psi to approx. 21 psi in 100% IPA.

The support film 12 is wider than the filter pad and is heat sealed or adhered to the film 17 of the filter pad 10 such that the aperture 12a is centrally located relative to the filter pad.

The film 17 of the filter pad 10 is heat sealed/adhered to the support film 12 such that a circular non-sealed or non-adhered area of the filter pad larger than the 5mm aperture 12a is centrally located around the aperture 12a. The barrier membrane 11 is arranged over the filter pad 10 on the opposite side and is heat sealed/adhered to the support film 12 so that the filter pad 10 is enclosed in a package comprising on one side a gas permeable, liquid impermeable membrane 11 and on the other side a heat sealable PE support film 12 with a central venting aperture 12a.

The filter package may thereafter be attached to the bag wall 2 in which a venting aperture 8 smaller than aperture 12a has been punched. The attachment to the inner surface of the wall 2 may either be accomplished by rim welding/heat sealing of the support film 12/membrane 11 to the wall 2 at 13 or by adhering the package to said wall 2 with a suitable adhesive.

The UPE membrane 11 has a smooth, soft surface and the surface tension has been measured to be 36 mN/m according to ISO 8296 (1987), while contact angle measurements according to the Wihelmy Plate Technique show that the surface of the membrane is similar to Teflon as regards friction. Therefore the ability of the membrane surface to repel liquid is such that a film of liquid preventing gas from passing through the membrane is not formed on the surface thereof. The pore volume of the membrane material of 70-80% and the structure (see Figs. 12-16) with very narrow passages only allowing particles smaller than 0.45 micrometres to pass therethrough entails that the flatus gas easily may pass through while human intestinal or fecal bacteria in the flatus gas or feces are retained by the membrane 11.

Furthermore, liquids are prevented from flowing through the membrane. Laboratory measurements where the membrane was loaded with 700 mm water column showed that no water flowed through, and no droplets were formed on the surface even after a test duration of 8 days.

An Artificial Stool Test (Applicants' test method) consisting in filling bags 1 with artificial stool and inverting the bags so that the filter assembly was covered by artificial stool during 24 hours while observing whether artificial stool passed through the membrane gave excellent results. The artificial stool is composed of beans, salts, intestinal enzymes and other substances equivalent to the contents of the human intestine such that the test simulates the influences on the membrane in practice.

The barrier membrane 11 has a back pressure of approx. 2-3 mBar with an air flow therethrough of 100 ml/min at a pressure of 0.1 Bar.

Referring now to Fig. 3, the gas flow through the filter assembly is indicated by means of arrows, the gas flowing through the membrane 11 and into the filter pad 10 from the periphery thereof towards the centre thereof and out through the aperture 8 in the wall 2.

Referring now to Figs. 4-5, an alternative arrangement of the filter assembly on the outer surface of the wall 2 is shown, the same references indicating the same elements as in Fig. 2. The filter pad 10 is adhered to the outer surface of the wall centrally located around the venting aperture 8. A barrier membrane 14 of UPE material identical to the material of barrier membrane 11 in Fig. 2 is heat sealed or welded with the support film 12 to the inner surface of the wall 2. The filter pad 10 is covered by a protection membrane 15 of a gas permeable, liquid impermeable material that may be the same UPE material as the material of the membrane 14 or any other suitable material. The flow of flatus gasses through the filter assembly is indicated by the arrows in Fig. 5.

Referring now to Fig. 6, another alternative arrangement of a filter assembly combining the examples of Figs. 2 and 4 is shown.

It will be obvious to those skilled in the art that other filter designs may be employed and that the location and number of filter pads may be varied relative to the venting aperture. Furthermore, several venting apertures may be arranged in the wall 2 combined with one or more filter pads.

Referring now again to Figs 7-10, various embodiments of spacing means arranged between the filter pad 10 and the barrier membrane 11 are illustrated, the spacing means ensuring a gas flow space between the filter pad 10 and the barrier membrane 11. In Fig. 7 ridges or peaks 20 are formed in the surface of the film 18 or applied thereto. In Fig. 8 ridges or peaks 21 are formed in the surface of the barrier membrane 11 or applied thereto. In Fig. 9 filaments or spheres 22 are adhered to either the film 18 or the membrane 11. In Fig. 10 a layer of open cell foam material is adhered to either the film 18 or the membrane 11. In all embodiments it is obtained that substantially the entire area of the barrier membrane 11 is available for flow of flatus gasses to the filter pad 10.

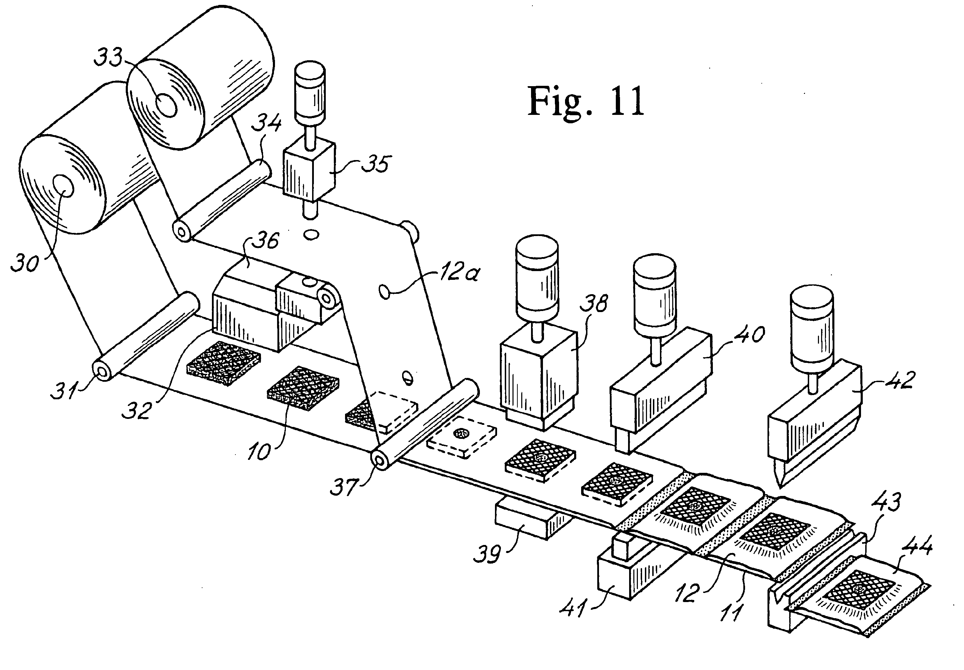

Referring now to Fig. 11, an apparatus for producing a filter assembly according to the invention and as shown in Fig. 2 is shown schematically and diagrammatically.

A supply roll 30 of the barrier membrane 11 feeds the membrane in a continuous web over an idler roller 31 below a locating device 32 for locating filter pads 10 on the membrane web with a predetermined spacing. A second supply roll 33 of the support film 12 feeds the support film in a continuous web over an idler roller 34 below a punching device 35, 36 for punching holes 12a in the support film spaced corresponding to the spacing of the filter pads 10. The punched support film continues over an idler roller 37 such that the support film is superimposed on the membrane 11 and the filter pads 10 with the holes 12a centrally located relative to the pads 10.

A heat sealing device 38, 39 heat seals the support film 12 to the gas impermeable film 17 of the filter pad 10 and thereafter a heat sealing device 40, 41 heat seals the support film 12 to the membrane 11 whereafter a cutting device 42, 43 cuts the combined support film 12 and membrane 11 in the middle of the heat seal performed by the device 42, 43 such that individual filter assembly packages 44 are formed ready for being attached to the wall 2 of the bag 1.

The heat sealing is carried out carefully to avoid compression of the relatively brittle filter cloth, so as to avoid crushing same. The pressure exerted by the device 38, 39 is only 4 kg at 3-4 Bar corresponding to a total filter welding pressure of only 0.15 - 0.20 N/mm² (filter surface).

It should be understood that while the shown and described pouch 1 is entirely closed by a total welding or heat sealing around the entire periphery, the pouch may also be of the type having a drainage aperture provided by not welding the lower edge regions at the two bag sheets to each other.

## Claims

1. A filter/venting assembly for use in a bag or pouch (1) for receiving discharge from the human body, said bag comprising:
- a distal wall (2) and a proximal wall (3), each of a gas and liquid impermeable, heat sealable or weldable thermoplastic material and having the edges thereof sealed together and defining the interior of the bag between the walls, the proximal wall having a stomal aperture (5), and one of the walls having a gas venting aperture (8) spaced from the stomal aperture,
- the filter/venting assembly for de-odorizing gas vented through the gas venting aperture being adapted for being arranged such that all the gas exiting the bag through the venting aperture flows through a filter element of the filter/venting assembly,
the filter/venting assembly comprising:
- an intermediate barrier membrane (11) arranged intermediate the filter element and the interior of the bag such that all gas exiting the bag through the filter element flows through the barrier membrane and for preventing liquids and solids in the interior of the bag from contacting the filter element while allowing gas to flow through the barrier membrane from the interior of the bag to the filter element,
- the filter element comprising a pad (10), a first surface of the pad facing the bar-rier membrane being covered by a gas impermeable film (18), preferably bonded thereto, such that the gas flow path through the filter element pad extends to or from the circumferential edge thereof,
**characterized in that** and
- the barrier membrane comprises an unlaminated microporous film of ultra high molecular weight polyethylene
- the filter element pad comprises at least two mutually superimposed layers of textile being highly, preferably nearly 100%, carbonized, each layer being bonded to an adjacent layer by means of a gas permeable adhesive film, preferably of EVA.

2. An assembly according to claim 1, wherein said microporous film has a porosity substantially only allowing passage through the barrier membrane of particles in said gas flow having a maximum dimension smaller than 2 micrometres, preferably 1.9 micrometres, more preferably 1.8 micrometres, even more preferably 1.7 micrometres, further preferably 1.6 micrometres, further preferably 1.5 micrometres, further preferably 1.4 micrometres, further preferably 1.3 micrometres, further preferably 1.2 micrometres, further preferably 1.1 micrometres, further preferably 1.0 micrometres, further preferably 0.9 micrometres, further preferably 0.8 micrometres, further preferably 0.7 micrometres, further preferably 0.6 micrometres, further preferably 0.5 micrometres.

3. An assembly according to claim 1, wherein said microporous film has a porosity substantially only allowing passage through the:barrier membrane of particles in said gas flow having a maximum dimension smaller than 0.45 micrometres.

4. An assembly according to claim 1, wherein said microporous film has a porosity substantially only allowing passage through the barrier membrane of particles in said gas flow having a maximum dimension smaller than 0.3 micrometres, preferably smaller than 0.2 micrometres.

5. An assembly according to claim 1, wherein said microporous film has a porosity allowing retention by the barrier membrane of substantially all human intestinal or fecal bacteria in said gas flow.

6. An assembly according to claim 1, wherein said microporous film has a porosity allowing retention by the barrier membrane of substantially all human intestinal or fecal viruses in said gas flow.

7. An assembly according to any of the preceding claims, wherein spacing means are arranged between the filter element and the barrier membrane so as to ensure a space therebetween for allowing a flow of gas in said space.

## Patentansprüche

1. Entlüftungs-/Filteranordnung zum Gebrauch in einem Beutel oder Sack (1) zum Aufnehmen von Ausscheidungen aus dem menschlichen Körper, wobei der Beutel umfasst:
- eine distale Wand (2) und eine proximale Wand (3), jede aus einem gas- und flüssigkeitsundurchlässigen, heißsiegelbaren or schweißbaren thermoplastischen Material, wobei die Wände miteinander schließende Kanten aufweisen, die den Innenraum des Beutels zwischen den Wänden definieren, wobei die proximale Wand eine Stomaöffnung (5) aufweist, und eine der Wände eine im Abstand zur Stomaöffnung angeordnete Gasentlüftungsöffnung (8) aufweist,
- wobei die Entlüftungs-/Filteranordnung zum Desodorieren von Gas, das durch die Gasentlüftungsöffnung entlüftet ist, dazu eingerichtet ist, so angeordnet zu werden, dass alles Gas, das durch die Entlüftungsöffnung den Beutel verlässt, durch ein Filterelement der Entlüftungs-/Filteranordnung strömt,
wobei die Entlüftungs-/Filteranordnung umfasst:
- eine Zwischensperrmembrane (11), die zwischen dem Filterelement und dem Innenraum des Beutels so angeordnet ist, dass alles Gas, das durch das Filterelement den Beutel verlässt, durch die Sperrmembrane strömt, und um Flüssigkeiten und Festkörper im Innenraum des Beutels daran zu hindern, das Filterelement zu berühren, während Gas ermöglicht wird, durch die Sperrmembrane aus dem Innenraum des Beutels zum Filterelement zu strömen,
- wobei das Filterelement ein Kissen (10) umfasst, wobei eine erste Oberfläche des Kissens, die der Sperrmembrane gegenüberliegt, von einem gasundurchlässigen Film (18) abgedeckt, vorzugsweise daran geklebt ist, so dass der Gasströmungsweg durch das Filterelementkissen sich bis zu oder von dessen peripherer Kante erstreckt,
**dadurch gekennzeichnet, dass**
- die Sperrmembrane eine unlaminierte mikroporöse Folie aus Polyethylen mit extrem hohem Molekulargewicht umfasst, und
- das Filterelementkissen mindestens zwei gegenseitig übereinander angeordnete Schichten aus hoch-, vorzugsweise fast 100%, karbonisiertem Textil umfasst, wobei jede Schicht durch eine gasdurchlässige Klebfolie, vorzugsweise aus EVA, an einer benachbarten Schicht geklebt ist.

2. Anordnung nach Anspruch 1, wobei die mikroporöse Folie eine Porosität aufweist, die im Wesentlichen nur Durchtritt durch die Sperrmembrane von Partikeln in der Gasströmung ermöglicht, die eine maximale Dimension kleiner als 2 Mikrometer, vorzugsweise 1,9 Mikrometer, mehr vorzugsweise 1,8 Mikrometer, sogar mehr vorzugsweise 1,7 Mikrometer, ferner vorzugsweise 1,6 Mikrometer, ferner vorzugsweise 1,5 Mikrometer, ferner vorzugsweise 1,4 Mikrometer, ferner vorzugsweise 1,3 Mikrometer, ferner vorzugsweise 1,2 Mikrometer, ferner vorzugsweise 1,1 Mikrometer, ferner vorzugsweise 1,0 Mikrometer, ferner vorzugsweise 0,9 Mikrometer, ferner vorzugsweise 0,8 Mikrometer, ferner vorzugsweise 0,7 Mikrometer, ferner vorzugsweise 0,6 Mikrometer, vorzugsweise 0,5 Mikrometer aufweisen.

3. Anordnung nach Anspruch 1, wobei die mikroporöse Folie eine Porosität aufweist, die im Wesentlichen nur Durchtritt durch die Sperrmembrane von Partikeln in der Gasströmung ermöglicht, die eine maximale Dimension kleiner als 0,45 Mikrometer aufweisen.

4. Anordnung nach Anspruch 1, wobei die mikroporöse Folie eine Porosität aufweist, die im Wesentlichen nur Durchtritt durch die Sperrmembrane von Partikeln in der Gasströmung ermöglicht, die eine maximale Dimension kleiner als 0,3 Mikrometer, vorzugsweise kleiner als 0,2 Mikrometer, aufweisen.

5. Anordnung nach Anspruch 1, wobei die mikroporöse Folie eine Porosität aufweist, die Zurückhaltung im Wesentlichen aller menschlichen Darm- oder fäkalen Bakterien in der Gasströmung durch die Sperrmembrane ermöglicht.

6. Anordnung nach Anspruch 1, wobei die mikroporöse Folie eine Porosität aufweist, die Zurückhaltung im Wesentlichen aller menschlichen Darm- oder fäkalen Viren in der Gasströmung durch die Sperrmembrane ermöglicht.

7. Anordnung nach einem der vorhergehenden Ansprüche, wobei Abstandsmittel zwischen dem Filterelement und der Sperrmembrane angeordnet sind, um dazwischen einen Zwischenraum zu sichern, um im Zwischenraum eine Strömung von Gas zu ermöglichen.

## Revendications

1. Dispositif de dégazage et filtration pour usage dans une poche ou une pochette (1) destinée à recevoir des substances éliminées par le corps humain, ladite poche comprenant:
- une paroi éloignée (2) et une paroi proche (3), chacune d'une matière thermoplastique étant thermoscellable ou soudable et imperméable aux gaz et aux liquides, et dont les bords sont soudés l'un à l'autre et délimitent l'intérieur de la poche entre les parois, la paroi proche ayant une ouverture stomale (5), et l'une des parois ayant une ouverture pour le mise à l'air de gaz (8), laquelle ouverture est espacée de l'ouverture stomale,
- le dispositif de dégazage et filtration pour la désodorisation de gaz mise à l'air à travers l'ouverture de mise à l'air de gaz et étant adapté de telle manière que le gaz sortant de la poche à travers l'ouverture de mise à l'air coule à travers un élément filtrant du dispositif de dégazage et filtration,
le dispositif de dégazage et filtration comprenant:
- une membrane de barrière intermédiaire (11) arrangée entre l'élément filtrant et l'intérieur de la poche de telle manière que tout le gaz sortant de la poche à travers l'élément filtrant coule à travers la membrane d'étanchéité, et pour éviter que des liquides et des corps solides à l'intérieur de la poche entre en contact avec l'élément filtrant tout en permettant le gaz de couler à travers la membrane d'étanchéité à partir de l'intérieur de la poche jusqu'à l'élément filtrant,
- l'élément filtrant comprenant un tampon (10), une première surface du tampon face à la membrane d'étanchéité étant couverte d'un film imperméable au gaz (18), préférablement attaché là-dessus, de telle manière que la voie du courant de gaz à travers le tampon de l'élément filtrant s'étend au bord circonférentiel du tampon de l'élément filtrant, ou à partir du bord circonférentiel du tampon de l'élément filtrant,
**caractérisé en ce que**
- la membrane d'étanchéité comprend un film microporeux non-stratifié de polyéthylène de poids moléculaire ultra haut, et
- le tampon de l'élément filtrant comprend au moins deux couches mutuellement superimposées de textile étant hautement carbonisé, préférablement presque à 100%, chaque couche étant liée à une couche adjacente par moyens d'un film adhésif perméable au gaz, préférablement de EVA.

2. Dispositif selon la revendication 1, dans lequel ledit film microporeux a une porosité permettant essentiellement seulement le passage à travers la membrane d'étanchéité de particules dans ledit courant de gaz, ayant une dimension maximale en dessous de 2 micromètres, préférablement 1.9 micromètres, plus préférablement 1.8 micromètres, davantage 1.7 micromètres, plus davantage 1.6 micromètres, plus davantage 1.5 micromètres, plus davantage 1.4 micromètres, plus davantage 1.3 micromètres, plus davantage 1.2 micromètres, plus davantage 1.1 micromètres, plus davantage 1.0 micromètres, plus davantage 0.9 micromètres, plus davantage 0.8 micromètres, plus davantage 0.7 micromètres, plus davantage 0.6 micromètres, plus davantage 0.5 micromètres.

3. Dispositif selon la revendication 1, dans lequel ledit film microporeux a une porosité permettant essentiellement seulement le passage à travers la membrane d'étanchéité de particules dans ledit courant de gaz, ayant une dimension maximale en dessous de 0.45 micromètres.

4. Dispositif selon la revendication 1, dans lequel ledit film microporeux a une porosité permettant essentiellement seulement le passage à travers la membrane d'étanchéité de particules dans ledit courant de gaz, ayant une dimension maximale en dessous de 0.3 micromètres, préférablement en dessous de 0.2 micromètres.

5. Dispositif selon la revendication 1, dans lequel ledit film microporeux a une porosité permettant la rétention d'essentiellement toutes les bactéries intestinales ou fécales humaines dans ledit courant de gaz.

6. Dispositif selon la revendication 1, dans lequel ledit film microporeux a une porosité permettant la rétention d'essentiellement tous les virus intestinaux ou fécaux humains dans ledit courant de gaz.

7. Dispositif selon l'une quelconque des revendications précédentes, dans lequel les moyens d'espacement sont organisés entre l'élément filtrant et la membrane d'étanchéité afin d'assurer un espace entre les deux pour permettre la présence d'un courant de gaz dans ledit espace.
